# EUROPEAN PATENT SPECIFICATION

(11) **EP 4 333 777 B1**
(45) Date of publication and mention of the grant of the patent: **14.10.2026**
(21) Application number: 22798773.2
(22) Date of filing: 02.05.2022
(51) Int. Cl.: A61F 5/01, A61H 1/02, A63B 21/00, A63B 23/16, A63B 24/00, B25J 9/08, B25J 9/12

(54) **DYNAMIC ORTHOSIS**
DYNAMISCHE ORTHESE
ORTHÈSE DYNAMIQUE

(30) Priority: 03.05.2021 US 202163183106 P
(43) Date of publication of application: 13.03.2024
(73) Proprietor: Lifeward Ltd, 2069203 Yokneam (IL)
(72) Inventor: KATZ, Geva, 7914000 Kibbutz Zikim (IL); ZILZER, Idan, 1224500 Kibbutz Dan (IL)
(74) Representative: Bals & Vogel Patentanwälte PartGmbB
(86) International application number: PCT/IL2022/050454
(87) International publication number: WO 2022/234572

(56) References cited:
- JP-B2- 5 472 680
- US-A1- 2003 110 938
- US-A1- 2007 038 161
- US-A1- 2017 119 614
- US-A1- 2017 266 075
- US-A1- 2018 296 419
- US-A1- 2020 113 770
- US-A1- 2020 281 798
- US-A1- 2021 079 935
- US-A1- 2021 106 489

## Description

### RELATED APPLICATION

This application claims the benefit of priority of U.S. Provisional Patent Application No. 63/183,106 filed on May 3, 2021.

### BACKGROUND

The present invention relates to an orthosis and to methods of fitting and using same in hand rehabilitation. Embodiments of the present invention relate to a dynamic orthosis that includes one or more flexible elements that can be flexed via a rode/wire connected to a drive unit.

The American Stroke Association states that stroke is a leading cause of death and longterm disability caused by loss of motor skills among American adults. Studies have shown that intensive and repetitive training may be necessary to recover functional motor skills.

Hand impairment after stroke contributes substantially to disability in the United States and around the world. People who have suffered strokes often experience significant delays in how long it takes to grip and release objects. Clinical therapy can reduce hand impairment but issues such as cost and access to a therapist limit recovery. Home-based therapy carried out by following prescribed hand exercises can be effective but a lack of compliance and a high dropout rate limit success.

Numerous robotic devices for hand function rehabilitation with various levels of complexity and functionality have been developed over the last decade. These devices range from simple mechanisms that support single joint movements to mechanisms with as many as 18 degrees-of-freedom (DOF) that can support multi-joint movements at the wrist and fingers.

Such devices (termed herein dynamic orthosis) are commonly used in hand therapy to substitute for weak or absent muscles or to apply force to stiff tissue and joints to regain passive joint motion.

Although such devices can be effective and can increase compliance, a poor fit and use of too much force can cause injury resulting in pain and edema whereas too little force will not achieve the desired goals of the intervention.

There is thus a need for, and it would be highly advantageous to have, a dynamic orthosis devoid of the above limitations.

US 2021/0106489 A1, upon which the preamble of claim 1 is based, discloses an orthosis device for rehabilitation comprising a main housing assembly worn on an upper extremity with a motor mechanism to actuate finger movement, and a flexible intermediate structure having a plurality of baffle members with a pushing-and-pulling wire extending longitudinally therethrough. The wire operates to compress or expand the upper portion of the baffle members while a flat bottom structure maintains constant spacing at the lower portion, thereby causing flexion and extension motion of secured fingers.

US 2021/0079935 A discloses multi-segment soft actuators with reinforcement structures configured to produce different actuation motions (bending, extending, expansion, contraction, twisting) in different segments when fluid flows into or out of a chamber. The actuators can be applied to a wearable robotic glove for hand rehabilitation, with soft segmented actuators mounted on the dorsal side of the hand to mimic the curving function of fingers.

### SUMMARY

The technical solution of the present invention is provided by the features of the independent claims. Variations are as described by the dependent claims.

According to one aspect of the present invention there is provided a dynamic orthosis attachable to a hand of a subject, the orthosis including at least one flexible element attachable to digital bones spanning at least one bone joint, the flexible element being composed of a plurality of interconnected units, wherein when the flexible element is bent, a distance between the units at a top of the flexible element changes while remaining unchanged at the bottom of the flexible element.

According to embodiments of the present invention the flexible element has an accordion-like shape.

According to embodiments of the present invention each of the interconnected units is a V or U-shaped vertical column.

According to embodiments of the present invention the interconnected units are attached to a base plate running a length of the flexible element.

According to embodiments of the present invention the flexible element includes a longitudinal opening.

According to embodiments of the present invention the units are of varying height.

According to embodiments of the present invention the units decrease in height from one end of the flexible element to the opposite end.

According to embodiments of the present invention the at least one flexible element bends with the joint during bending of the digital bones.

According to embodiments of the present invention the dynamic orthosis comprises five flexible elements, each attachable to digital bones spanning a different bone joint.

According to embodiments of the present invention the dynamic orthosis further comprises a rod or wire running through the longitudinal opening.

According to embodiments of the present invention a distal end of the rod or wire is attached to a distal end of the flexible element.

According to embodiments of the present invention the dynamic orthosis further comprises a drive unit attached to a proximal end of the rod.

According to embodiments of the present invention the drive unit is configured for pulling the rod to thereby arc the flexible element.

According to embodiments of the present invention an arc length of a top of the flexible element increases when the rod is pulled with the arc length of the bottom remaining unchanged.

According to embodiments of the present invention the digital bones include metacarpals and/or phalanges.

According to embodiments of the present invention the at least one bone joint is a metacarpophalangeal and/or proximal or distal interphalangeal joint.

According to embodiments of the present invention the dynamic orthosis further comprises a sensor (e.g., hall sensor) for measuring a compression or tension of the rod or wire.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

Implementation of the method and system of the present invention involves performing or completing selected tasks or steps manually, automatically, or a combination thereof. Moreover, according to actual instrumentation and equipment of preferred embodiments of the method and system of the present invention, several selected steps could be implemented by hardware or by software on any operating system of any firmware or a combination thereof. For example, as hardware, selected steps of the invention could be implemented as a chip or a circuit. As software, selected steps of the invention could be implemented as a plurality of software instructions being executed by a computer using any suitable operating system. In any case, selected steps of the method and system of the invention could be described as being performed by a data processor, such as a computing platform for executing a plurality of instructions.

### BRIEF DESCRIPTION OF THE SEVERAL VIEWS OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIGs. 1A-D illustrate an embodiment of the flexible element of the present invention shown in linear (Figures 1A, B) and bent (Figures 1C, D) positions from the top (Figures 1A, C) and side (Figure 1B, D).
FIG. 1E illustrates another embodiment of the flexible element of the present invention in which a locking plate is fitted to the top of the columns to limit or reduce bending of that portion.
FIG. 2 illustrates the dynamic orthoses with five flexible elements attached to a drive unit through actuation rods/wires.
FIGs. 3A-B illustrates the flexible element and attached force sensor.
FIG. 4 illustrates the force sensor of the present orthoses.
FIG. 5 illustrates a prototype orthoses constructed in accordance with the teachings of the present invention.
FIG. 6 is a graph illustrating the mechanical and geometrical multiplication of force applied by a prototype orthoses constructed in accordance with the teachings of the present invention.
FIG. 7 is a graph illustrating the relationship between efficiency and cable bending in a prototype orthoses constructed in accordance with the teachings of the present invention.
FIG. 8 is a graph illustrating the efficiency of force transfer to the finger by a prototype orthoses constructed in accordance with the teachings of the present invention.
FIG. 9 is a graph illustrating the force produced by a prototype orthoses constructed in accordance with the teachings of the present invention resulting in a 10N bending force on the distal tip of the finger

### DETAILED DESCRIPTION

The present invention is of a dynamic orthosis and methods of making and using same in hand rehabilitation and training as well as an assist device for daily functions.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

Rehabilitation plays a crucial role in improving hand and finger function following stroke. Robotics and mechatronic devices have been adapted for use in rehabilitation and have increased the quality of lives of individuals with disabilities, offering dedicated training that can outperform conventional approaches.

Robotic devices are advantageous in that they can increase user compliance and can be used in a home setting. However, poor fitting devices can either cause injury resulting in pain and edema or can fail in achieving the desired goals of intervention.

While reducing the present invention to practice, the present inventors devised an orthotic device capable of providing a controllable range of motion while accurately tracking the flexion and extension of natural hand joints.

Thus, according to one aspect of the present invention there is provided a dynamic orthosis attachable to a hand of a subject.

In order to provide the range of motion (for each digit) required for therapy, the dynamic orthosis of the present invention includes a plurality of flexible elements (typically 4 or 5) each attachable to a specific digit of a hand. Alternatively, the dynamic orthosis of the present invention can include less than 5 flexible elements (1, 2, 3, 4) for providing therapy to select digits or for alternating therapy between digits (e.g., in the case of a single flexible element). The flexible element has an accordion-like shape capable of elastically bending between an arc-like configuration and a linear configuration while maintaining an alignment with a joint of the digit during bending of the digital bones.

To provide movement between these linear and arced configurations, the dynamic orthoses of the present invention includes one or more drive units (e.g., one for each flexible element) connected to a rod or wire running the length of the flexible element. Pushing and pulling the rod/wire flexes and extends the flexible element thereby providing the range of motion suitable for rehabilitation.

The flexible element includes a base plate onto which a plurality of interconnected V/U-shaped columns are attached to form the accordion like shape. When flexed (arced downward), the distance between the columns at the top of the flexible element increases while the distance at the bottom (at the point of attachment to the base plate) remains unchanged. Such a configuration does not produce any parallel (push/pull) forces on a digit during articulation and thus ensures that forces applied by the orthosis during rehabilitation training are directed at digit flexion/extension only. The latter is important since the amount of force applied to an individual's digit by the orthosis must fall within a specific range in order to avoid injury on the one hand and provide effective therapy on the other. A force applied along the length of the digit can result in injury to the joints and/or can diminish the bending force necessary for therapy thereby not achieving the desired therapeutic effect.

In order to maintain the force within a therapeutic range, the dynamic orthoses can include a compression and tension sensor to measure a force applied to the rod/wire to thereby determine the force applied to the digits and maintain it within a suitable range.

In order to enable the dynamic orthosis of the present invention to apply the desired bending forces to the individual's digits, the present invention also provides one or more motorized drive units that are connectable to the rod/wire of flexible elements (e.g., flexible elements of various lengths and/or number).

The dynamic orthosis of the present invention can be custom-fitted to an individual's hand by using hand-specific data (e.g., image, hand measurements) to produce (e.g., via 3D printing) flexible elements that are user-specific.

Following manufacturing and optionally fitting, one or more flexible elements can be connected to a drive unit and calibrated for use (using tension/compression sensor data). Each flexible element can then be attached to a digit (by, for example, Velcro loops or by attaching each element to a glove fitted to the user's hand). The drive unit can then be attached to the user's wrist or forearm using Velcro loops or straps.

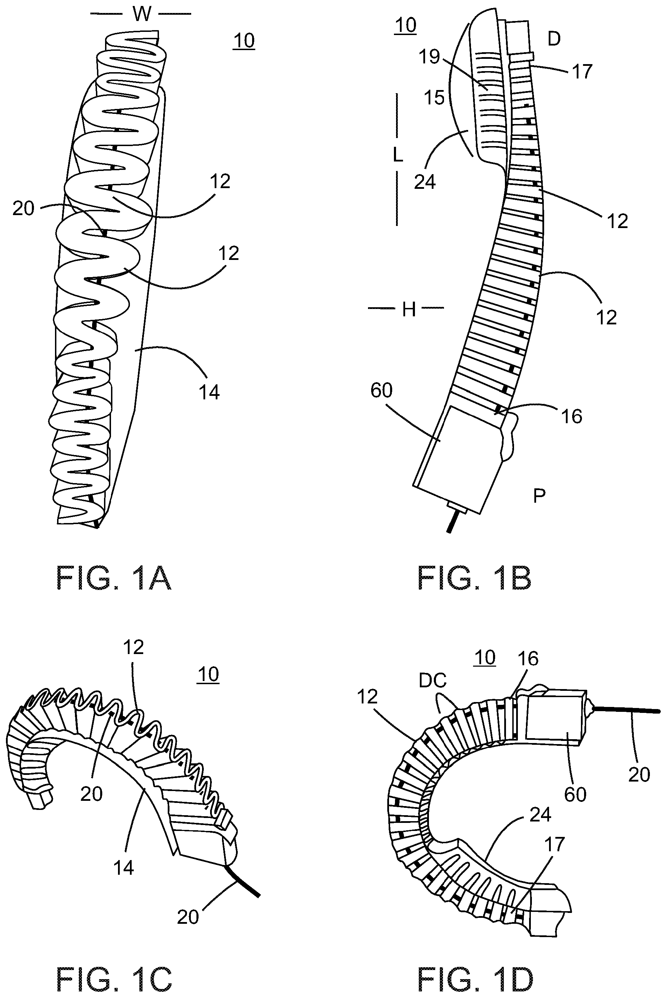

Referring now to the drawings, Figures 1A-D illustrate the flexible element of the present invention which is referred to herein as element 10.

Flexible element 10 can be 50-150 mm long (L), 3-20 mm in width (W) and 3-60 mm in height (H). Flexible element 10 can be fabricated from a polymer such as polyurethane, nylon or any other flexible compound, using injection molding or 3D printing techniques.

Flexible element includes a plurality of interconnected columns 12 (typically V or U-shaped) attached at the bottom (B) to a base plate 14. Columns 12 form a tortuous strip running the length of flexible element 10 (best see in Figure 1A). Any number of columns 12 can be used in flexible element 10. A typical number can be in the range of 10-50 depending on the overall length of flexible element 10 and that of each column 12. Base plate 14 can be flat or concave along its width to better fit the rounded contour of a top of a digit.

Columns 12 can vary in height from the proximal (P) end to the distal (D) end of the flexible element. For example, a proximal end column 16 can be 3-30 mm in height while a distal end column 17 can be 3-15 mm in height. Such a configuration is advantageous since it maintains a constant height of rod/wire 20 over base plate 14 and it provides support points to rod/wire 20 during bending thus preventing buckling/kinking of rod/wire 20 when pushed.

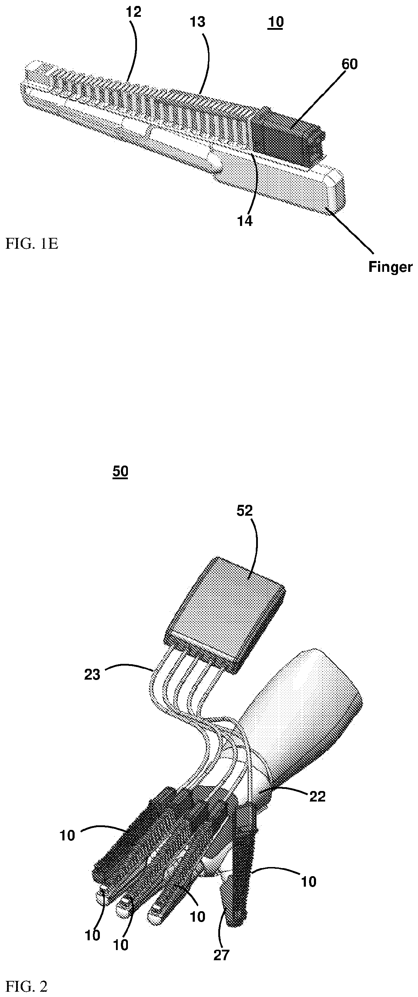

Portion 15 of flexible element 10 corresponding to an end portion of a digit (e.g., distal phalange) can be stiffened with an additional underlying row of ribs 19 that form a structure 24 that mounts over an end portions of a finger. For further stabilization, structure 24 can be overlaid with a sock 27 (Figure 2).

As is shown in Figures 1A-D, when flexed (Figure 1C, D), a distance between adjacent columns 12 (DC) increases at the top of flexible element 10 while remaining unchanged at the bottom of flexible element 10 (as compared to the linear configuration shown in Figures 1A, B). This ensures that when attached to a digit, flexion of flexible element 10 follows a center of articulation of the digit without producing any parallel forces on the digit (forces along the length of the digit). Flexible element 10 can have a range of motion from zero degrees (flat) up to 250 degrees (tip of distal phalanx to palm) and can fluidly flex throughout this range.

Flexible element 10 includes an opening running a length thereof near the top of columns 12. Such an opening can accommodate a push/pull rod/wire/ 20 that is attached to a distal end of flexible element. Pushing/pulling of rod/wire 20 flexes and extends flexible element 10 (respectively).

The V/U-shaped interconnected columns 12 maintain wire/rod 20 at a constant distance above base plate 14 thereby ensuring a constant bending movement while preventing collapse of wire/rod 20 under compression forces.

The bending movement of flexible element is induced by a change in length of the section of the metal rod at flexible element 10. Since rod/wire 20 slides through columns 12 relative to base plate 14 (that does not change in length), when wire/rod 20 is pushed, flexible element bends around base plate 14. The relative change in length of wire/rod 20 (spanning the top of flexible element 10) determines the overall angle of bending which is the sum of bending angles of the metacarpophalangeal (MP), proximal interphalangeal (PIP) and distal interphalangeal (DIP) joints of a digit.

The exact amount of bending for each separate joint is affected by the resistance to bending to the cross section, it can be affected by the human anatomy and can be manipulated using changes in column 12 heights.

During bending, rod/wire 20 experiences compression forces while base plate 14 is subjected to stretching forces. During extension, wire/rod 20 is subjected to tension while base plate 14 is subjected to compression.

Flexible element 10 can bend along its entire length or a portion thereof. For example, by interconnecting a top of columns 12 with a rigid (e.g., metal) or semi-flexible (e.g., rubber) locking plate 13 (e.g., Figure 1E), one can limit or reduce bending of a specific portion of flexible element 10 spanning a specific finger joint. Such a configuration can be used to provide therapy to one or two joints of a finger or to provide limited-movement therapy to one or more joints.

Alternative configuration for limiting bending of portions of flexible element 10 can include a leaf spring or rigid element attached to base plate 14 or in between columns 12.

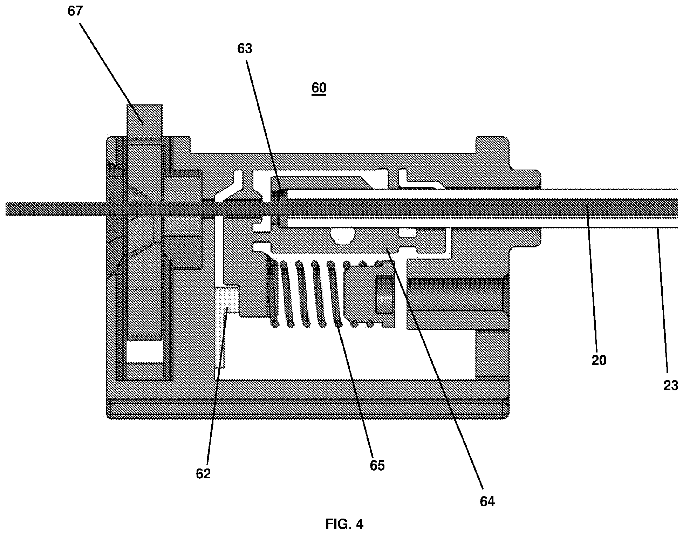

Figure 2 illustrates an orthoses 50 that includes 5 flexible elements attached to a drive unit 52 via wires/rods 20 (e.g., Teflon-coated cable). The portion of rod/wire 20 positioned outside of flexible element is covered by a sleeve 23 that abuts a wall 25 of a parallelogram structure 64 within sensor 60 (see Figure 4) in order to translate rod/wire 20 movement to movement of flexible element 10.

Drive unit 52 can include one or more motors or servos for pushing/pulling wire/rod 20 of each flexible element 10 to thereby flex/extend each flexible element 10.

Each flexible element 10 can be attached to a specific digit by covering structure 24 (also shown in Figure 1B) of flexible element 10 with a finger sock 27 and mounting sock 27 over an end portion of a finger and attaching the proximal portion of flexible element 10 to a half glove 22 via Velcro fasteners and the like.

Orthoses 50 can further include a data storage unit, a CPU and a communications module. The data collected by sensor 60 can be stored in orthoses 50 and used to calculate a subject's preferred force range (force applied to rod/wire 20). Such calculation can be performed by the CPU of orthoses 50 or by a connected device (e.g., Smartphone) communicating with orthoses 50. The preferred force range, therapy sessions and any other parameters related to device operation can be stored on orthoses 50, on the user's device or in the cloud.

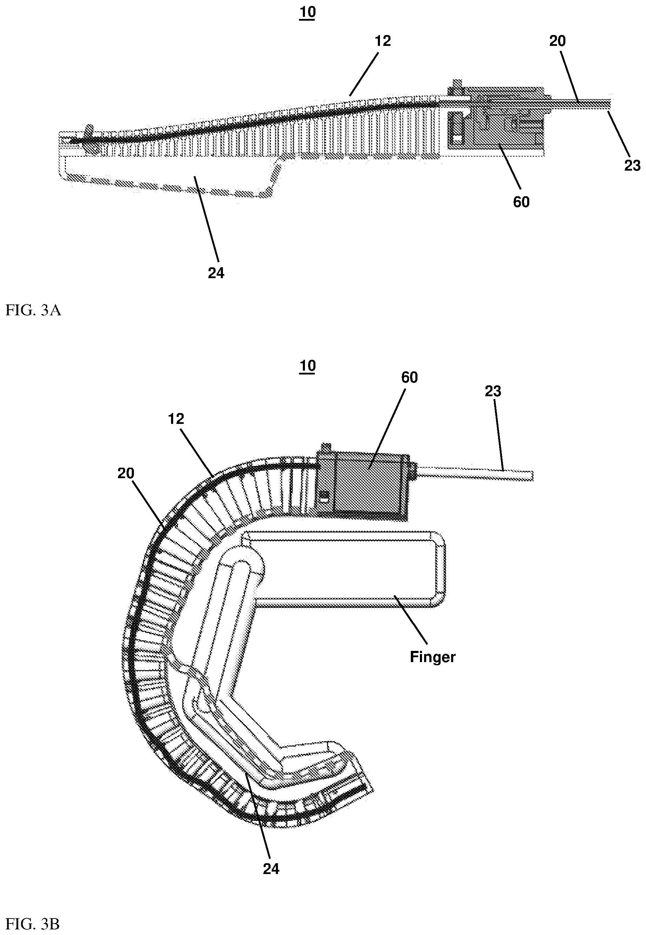

Figures 3A-B illustrate the position of base plate 14 and rod/wire 20 in an extended flexible element 10 (Figure 3A) and flexed flexible element 10 (Figure 3B) when attached to a user's hand using half-glove 22 and finger caps/socks 24.

The forces applied to the digit by wire/rod 20 and base plate 14 are shown by the solid and dashed lines respectively.

In order to calibrate the force applied by each flexible element 10 to a respective digit, flexible element 10 can include a force sensor 60 (Figures 1E, 2 and 3) for measuring a tension and compression on rod/wire 20.

Figure 4 illustrates force sensor 60 in greater detail. Sensor 60 is attached to a proximal end of flexible element 10 (not shown). Wire/rod 20 runs through sleeve 23 that abuts stop 63 of sensor 60 and the compression and tension forces applied to wire/rod 20 with respect to flexible element 10 are measured by a Bowden system conductor 62. Based on Newton's third law, at a specific cross section the sum of all forces must be zero and as such, the rod exerts the same force in the opposite direction as that measured by sensor 60.

A parallelogram mechanism 64 is used in sensor 60 to apply resistance to any 'bending moments' in the Bowden conductor itself thereby ensuring that sensor 60 only measures compression and tension on wire/rod 20 (via sleeve 23). Since sensor 60 can measure compression, a spring 65 is used in order to enable measurement of both compression and tension by providing a preload force on the sensor. The measured forces are opposite to the forces on rod/wire 20, when rod/wire 20 is under tension sensor measurement is "preload force (spring) combined with tension, when rod/wire 20 is under compression sensor measurement is "preload force (spring) combined with compression. Element 67 is a latch for securing and releasing sensor 60 from flexible element 10.

The relationship between the force applied to wire/rod 20 and the force exerted by a human finger changes throughout finger flexion and is affected by the height of the rod/wire 20 above base plate 14 and the length of the finger as well as frictional coefficients between the wire/rod 20 and sleeve 23 and the wire/rod 20 and flexible element 10 (see Example 2 for further detail).

Since a specific human finger can resist flexion, and since flexible element 10 is elastic and will also resist flexion, calibration of orthoses 50 prior to treatment will provide the most accurate indication of forces applied to the fingers.

Calibration is accomplished by flexing and extending a finger several times, while instructing the subject not to resist movement of the finger. The forces applied to wire/rod 20 as measured by sensor 60 can then be analyzed and a compensation table of measured forces along bending movement can then be constructed and used to extract the actual forces between orthoses 50 and the human finger during its operation.

Such fitting and calibration can be conducted by the subject and monitored by a technician or therapist over a communication network.

Once clinical calibration is complete the parameters can be stored on orthoses 50, the cloud or a user's device.

When used at home in therapy (as part of ADL), the device will run through a self-check by moving each finger and referencing angle position sensors, force sensors the motor position to evaluate device operation.

The present orthoses can be used for therapy as Motor Priming. Priming is a type of implicit learning wherein a stimulus prompts a change in behavior (Stinear et al. Priming the motor system enhances the effects of upper limb therapy in chronic stroke. Brain, a journal of neurology, volume 131 issue 5 year 2008). For example, priming of the motor cortex is associated with changes in neuroplasticity that are associated with improvements in motor performance. In general, the technique is to move the body part with repetitive movements through fixed timing to create a repetitive loop. Priming is an emerging paradigm/trends in rehabilitation since it improves the overall recovery while reducing 30% of traditional treatment time, thus significantly improving the productivity of the clinic.

The present orthoses can automate priming treatments in, for example, stroke and spinal cord injury patients.

A typical treatment regimen can include the following:
- A protocol for improving patient grasp of objects: Straighten fingers to start operation (0 degrees)
- Close all fingers at once with passive movement (TPM) to 200 degrees or more.
- Straighten fingers to start operation (0 degrees)
- Duration of work should be configured based on the state of the patient from 10 seconds to 2, no stops open->close->open->close.

Repeat the procedure for 15 minutes than continue standard treatment for only 30 minutes (normal treatment - without priming is 45 min).

As used herein the term "about" refers to ± 10 %.

Additional objects, advantages, and novel features of the present invention will become apparent to one ordinarily skilled in the art upon examination of the following examples, which are not intended to be limiting.

### EXAMPLES

Reference is now made to the following examples, which together with the above descriptions, illustrate the invention in a non-limiting fashion.

### EXAMPLE 1

### Prototype Construction and Testing

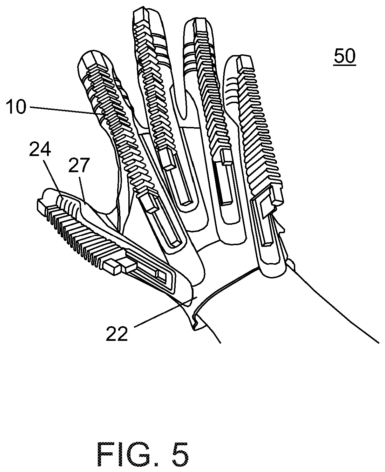

A prototype of the present orthoses was constructed and tested on a subject's hand (Figure 5).

Flexible elements 10 were 3D printed using PCTPE - a combination of thermoplastic elastomer and nylon for resilience, good elongation (up to 400%) and low friction coefficient (measured at 0.27 between the PCPTE and the stainless steel cable).

Finger socks 27 were stitched from a stretchable fabric. For a proper connection to the PCPTE material, flexible elements 10 were printed directly onto finger socks 27 and over a Velcro fastener using a specially designed jig resulting in a firm bond between sock 27 and flexible element 10.

The mechanism of sensor 60 was printed from stiff acrylic material the sensor is glued in position and a preload spring 65 (Figure 4) is used for creating a preload force as described hereinabove.

Half glove 22 was stitched from a stretchable fabric and covered by a stretchable Velcro fastener.

Flexible elements were connected to the drive unit through a Bowden cable system.

Orthoses 50 was tested on several subjects, flexible elements functioned properly and developed a significant amount of force without any failure and achieved maximal flexion of fingers.

Orthoses 50 was also tested by a paralyzed subjected who managed to mount the orthoses on a paralyzed hand using the healthy hand in under two minutes.

### EXAMPLE 2

### Force Calculations

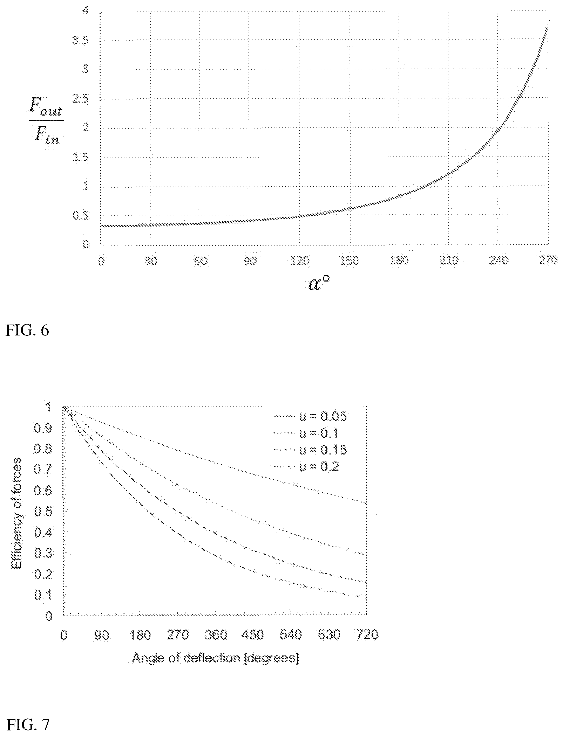

The prototype of Example 1 was further tested in order to determine the forces applied to a human finger. To that end, a the mechanical and geometrical multiplication of force applied by the prototype to a human finger was first determined and is presented in the graph of Figure 6

The losses of force in the prototype were determined by following:
(i) symmetry between push and pull of the cable (wire/rod);
(ii) dynamic friction coefficient calculation; and
(iii) elastic resistance of the cable to bending (minimal, measured as 1N for 180 degree bend).

The friction in the cable (mainly between the cable and outer sleeve at the segment of cable positioned outside the flexible element) is determined by the rigidity of the cable, the cable and sleeve materials, the velocity of the cable movement within the sleeve and torqueing forces applied to the cable by the drive unit.

The largest factors affecting friction are the friction coefficient (between cable and sleeve) and the toque applied to the cable.

These factors can be represented by: $\frac{{F}_{out}}{{F}_{in}} = {e}^{μ θ}$ (1)

In the prototype tested, the drive unit was located at the shoulder area of the tested subject and as such, the maximum bend on the cable (in the region between the drive unit and flexible element) when the elbow of the tested subject was bent was 120 degrees. A stainless steel cable surrounded by a PTFE sleeve (CoF of 0.2) resulted in a 65% efficiency of force transfer when the elbow was completely bent.

The relationship between efficiency and cable bending is presented in Figure 7.

The friction at the region of the flexible element between the stainless steel cable and the opening in the vertical columns of the flexible element is estimated at around 0.4.

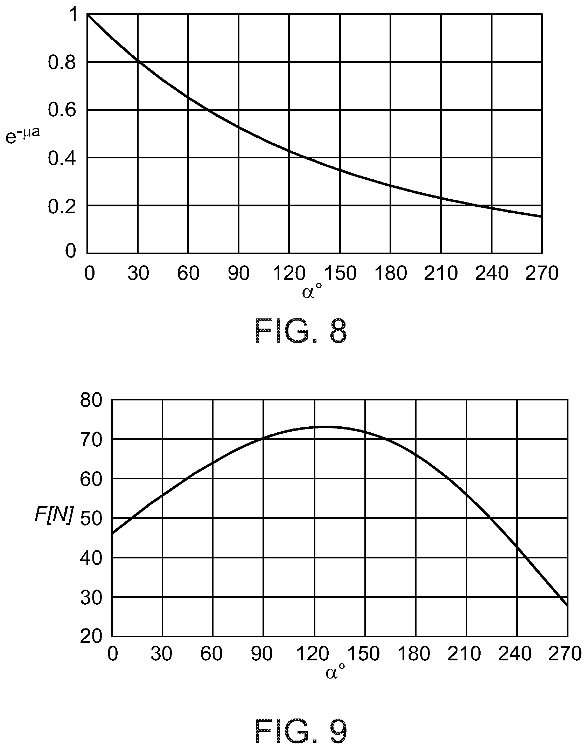

Taking these parameters into account, the efficiency of force transfer to the finger by the present prototype as tested is represented in the graph of Figure 8.

The force on the cable that is required for producing a force of 10N on the distal tip of the finger was calculated using the force multiplier produced by the prototype and the losses incurred by friction.

Such a force is presented in the graph of Figure 9 as a function of bending of arm and finger. As is shown by this graph, the maximal force applied to the cable (by the drive unit) necessary to achieve a bend of approximately 120 degrees (at the finger) is 75N and is well within the capabilities of a standard motor or servo drive unit.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art.

## Claims

1. A dynamic orthosis attachable to a hand of a subject, the dynamic orthosis including at least one flexible element (10) attachable to finger bones of a finger of the hand, the flexible element (10) comprising: a base plate (14), extending between a proximal end and a distal end of the flexible element (10); a plurality of interconnected units (12), coupled to the base plate (14) and extending along a length of the flexible element (10), each interconnected unit (12) defining a height between a top and a bottom of the flexible element (10); and a rod or wire (20), extending through a longitudinal opening of the interconnected units (12) along the length of the flexible element (10), wherein the flexible element (10) is configured to elastically bend between an arced configuration and a linear configuration, in alignment with a joint of the finger, wherein a flexion of the flexible element (10) is induced by a change in length of the rod or wire (20) at the flexible element (10) and when the flexible element (10) is flexed, a distance between the interconnected units (12) at the top of the flexible element (10) increases while remaining unchanged at the bottom of the flexible element (10), wherein the interconnected units (12) vary in height along at least a portion of the length of the flexible element (10), **characterized in that** the flexible element (10) is configured to be attached to a single finger of the hand, and the flexible element (10) spans a plurality of joints of the single finger.

2. The dynamic orthosis of claim 1, wherein the flexible element (10) has an accordion-like shape.

3. The dynamic orthosis of claim 1, wherein each of the interconnected units (12) is a V or U-shaped vertical column.

4. The dynamic orthosis of claim 1, wherein the interconnected units (12) decrease in height from one end of the flexible element (10) to an opposite end.

5. The dynamic orthosis of claim 1, wherein the flexible element (10) is configured to bend with the joint during bending of the finger bones.

6. The dynamic orthosis of claim 1, comprising five flexible elements (10), each attachable to finger bones of a respective finger of the hand.

7. The dynamic orthosis of claim 1, wherein a distal end of the rod or wire (20) is attached to a distal end of the flexible element (10).

8. The dynamic orthosis of claim 7, further comprising a drive unit (52) attached to a proximal end of the rod or wire (20), wherein the drive unit (52) is configured to push the rod or wire (20) to flex the flexible element (10), and to pull the rod or wire (20) to extend the flexible element (10).

9. The dynamic orthosis of claim 8, wherein an arc length of the top of the flexible element (10) increases when the rod or wire (20) is pushed while an arc length of the bottom of the flexible element (10) remains unchanged.

10. The dynamic orthosis of claim 1, wherein the finger bones include metacarpals or phalanges, and wherein the plurality ofj oints include a metacarpophalangeal and/or proximal or distal interphalangeal joint.

11. The dynamic orthosis of claim 1, further comprising a sensor (60), configured to measure a compression or tension of the rod or wire (20).

12. The dynamic orthosis of claim 11, wherein the sensor (60) comprises:
a Bowden conductor (62), configured for measuring compression and tension forces applied to the rod or wire (20) with respect to the flexible element (10); and
a parallelogram structure (64), configured to apply resistance to bending movements in the Bowden conductor (62).

13. The dynamic orthosis of claim 1, wherein the flexible element (10) comprises a locking plate (13), configured to interconnect a plurality of the interconnected units (12) for limiting or reducing a bending of a portion of the flexible element (10).

14. The dynamic orthosis of claim 1, wherein said flexible element (10) has a range of motion from zero degrees to 250 degrees.

15. The dynamic orthosis of claim 1, wherein a portion (15) of the flexible element (10) comprises an underlying row of ribs (19) that form a structure (24) that mounts over an end portion of the finger.

## Patentansprüche

1. Dynamische Orthese, befestigbar an einer Hand eines Subjekts, wobei die dynamische Orthese wenigstens ein flexibles Element (10) umfasst, befestigbar an Fingerknochen eines Fingers der Hand, wobei das flexible Element (10) umfasst:
eine Grundplatte (14), die sich zwischen einem proximalen Ende und einem distalen Ende des flexiblen Elements (10) erstreckt;
eine Mehrzahl miteinander verbundener Einheiten (12), die mit der Grundplatte (14) gekoppelt sind und sich entlang einer Länge des flexiblen Elements (10) erstrecken, wobei jede miteinander verbundene Einheit (12) eine Höhe zwischen einer Oberseite und einer Unterseite des flexiblen Elements (10) definiert; und
einen Stab oder Draht (20), der sich durch eine längliche Öffnung der miteinander verbundenen Einheiten (12) entlang der Länge des flexiblen Elements (10) erstreckt, wobei das flexible Element (10) dazu ausgebildet ist, sich elastisch zwischen einer bogenförmigen Konfiguration und einer linearen Konfiguration zu biegen, in Ausrichtung mit einem Gelenk des Fingers, wobei eine Beugung des flexiblen Elements (10) durch eine Längenänderung des Stabs oder Drahts (20) an dem flexiblen Element (10) hervorgerufen wird und, wenn das flexible Element (10) gebeugt ist, ein Abstand zwischen den miteinander verbundenen Einheiten (12) an der Oberseite des flexiblen Elements (10) zunimmt, während er an der Unterseite des flexiblen Elements (10) unverändert bleibt, wobei die miteinander verbundenen Einheiten (12) in der Höhe entlang wenigstens eines Abschnitts der Länge des flexiblen Elements (10) variieren, **dadurch gekennzeichnet, dass** das flexible Element (10) dazu ausgebildet ist, an einem einzelnen Finger der Hand befestigt zu werden, und das flexible Element (10) eine Mehrzahl von Gelenken des einzelnen Fingers überspannt.

2. Dynamische Orthese nach Anspruch 1, wobei das flexible Element (10) eine ziehharmonikaartige Form aufweist.

3. Dynamische Orthese nach Anspruch 1, wobei jede der miteinander verbundenen Einheiten (12) eine V- oder U-förmige vertikale Säule ist.

4. Dynamische Orthese nach Anspruch 1, wobei die miteinander verbundenen Einheiten (12) in der Höhe von einem Ende des flexiblen Elements (10) zu einem gegenüberliegenden Ende abnehmen.

5. Dynamische Orthese nach Anspruch 1, wobei das flexible Element (10) dazu ausgebildet ist, sich mit dem Gelenk während einer Beugung der Fingerknochen zu biegen.

6. Dynamische Orthese nach Anspruch 1, umfassend fünf flexible Elemente (10), jedes befestigbar an Fingerknochen eines jeweiligen Fingers der Hand.

7. Dynamische Orthese nach Anspruch 1, wobei ein distales Ende des Stabs oder Drahts (20) an einem distalen Ende des flexiblen Elements (10) befestigt ist.

8. Dynamische Orthese nach Anspruch 7, ferner umfassend eine Antriebsvorrichtung (52), die an einem proximalen Ende des Stabs oder Drahts (20) befestigt ist, wobei die Antriebsvorrichtung (52) dazu ausgebildet ist, den Stab oder Draht (20) zu schieben, um das flexible Element (10) zu beugen, und den Stab oder Draht (20) zu ziehen, um das flexible Element (10) zu strecken.

9. Dynamische Orthese nach Anspruch 8, wobei eine Bogenlänge der Oberseite des flexiblen Elements (10) zunimmt, wenn der Stab oder Draht (20) geschoben wird, während eine Bogenlänge der Unterseite des flexiblen Elements (10) unverändert bleibt.

10. Dynamische Orthese nach Anspruch 1, wobei die Fingerknochen Metakarpalknochen oder Phalangen umfassen und wobei die Mehrzahl von Gelenken ein Metakarpophalangealgelenk und/oder ein proximales oder distales Interphalangealgelenk umfasst.

11. Dynamische Orthese nach Anspruch 1, ferner umfassend einen Sensor (60), der dazu ausgebildet ist, eine Kompression oder Spannung des Stabs oder Drahts (20) zu messen.

12. Dynamische Orthese nach Anspruch 11, wobei der Sensor (60) umfasst:
einen Bowden-Leiter (62), der dazu ausgebildet ist, Kompressions- und Zugkräfte zu messen, die auf den Stab oder Draht (20) in Bezug auf das flexible Element (10) ausgeübt werden; und
eine Parallelogrammstruktur (64), die dazu ausgebildet ist, Widerstand gegen Biegebewegungen in dem Bowden-Leiter (62) aufzubringen.

13. Dynamische Orthese nach Anspruch 1, wobei das flexible Element (10) eine Verriegelungsplatte (13) umfasst, die dazu ausgebildet ist, eine Mehrzahl der miteinander verbundenen Einheiten (12) miteinander zu verbinden, um ein Biegen eines Abschnitts des flexiblen Elements (10) zu begrenzen oder zu reduzieren.

14. Dynamische Orthese nach Anspruch 1, wobei das flexible Element (10) einen Bewegungsumfang von null Grad bis 250 Grad aufweist.

15. Dynamische Orthese nach Anspruch 1, wobei ein Abschnitt (15) des flexiblen Elements (10) eine darunterliegende Reihe von Rippen (19) umfasst, die eine Struktur (24) bilden, die über einen Endabschnitt des Fingers montiert wird.

## Revendications

1. Orthèse dynamique pouvant être fixée à une main d'un sujet, l'orthèse dynamique comprenant au moins un élément flexible (10) pouvant être fixé à des os d'un doigt de la main, l'élément flexible (10) comprenant :
une plaque de base (14), s'étendant entre une extrémité proximale et une extrémité distale de l'élément flexible (10) ;
une pluralité d'unités interconnectées (12), couplées à la plaque de base (14) et s'étendant le long d'une longueur de l'élément flexible (10), chaque unité interconnectée (12) définissant une hauteur entre un sommet et un fond de l'élément flexible (10) ; et
une tige ou un fil (20), s'étendant à travers une ouverture longitudinale des unités interconnectées (12) le long de la longueur de l'élément flexible (10), dans laquelle l'élément flexible (10) est configuré pour se plier élastiquement entre une configuration arquée et une configuration linéaire, en alignement avec une articulation du doigt, dans laquelle une flexion de l'élément flexible (10) est induite par une variation de longueur de la tige ou du fil (20) au niveau de l'élément flexible (10) et, lorsque l'élément flexible (10) est fléchi, une distance entre les unités interconnectées (12) au sommet de l'élément flexible (10) augmente tout en restant inchangée au fond de l'élément flexible (10), dans laquelle les unités interconnectées (12) varient en hauteur le long d'au moins une partie de la longueur de l'élément flexible (10), **caractérisée en ce que** l'élément flexible (10) est configuré pour être fixé à un seul doigt de la main, et l'élément flexible (10) s'étend sur une pluralité d'articulations du seul doigt.

2. Orthèse dynamique selon la revendication 1, dans laquelle l'élément flexible (10) présente une forme en accordéon.

3. Orthèse dynamique selon la revendication 1, dans laquelle chacune des unités interconnectées (12) est une colonne verticale en forme de V ou de U.

4. Orthèse dynamique selon la revendication 1, dans laquelle les unités interconnectées (12) diminuent en hauteur d'une extrémité de l'élément flexible (10) à une extrémité opposée.

5. Orthèse dynamique selon la revendication 1, dans laquelle l'élément flexible (10) est configuré pour se plier avec l'articulation lors du pliage des os du doigt.

6. Orthèse dynamique selon la revendication 1, comprenant cinq éléments flexibles (10), chacun pouvant être fixé à des os d'un doigt respectif de la main.

7. Orthèse dynamique selon la revendication 1, dans laquelle une extrémité distale de la tige ou du fil (20) est fixée à une extrémité distale de l'élément flexible (10).

8. Orthèse dynamique selon la revendication 7, comprenant en outre une unité d'entraînement (52) fixée à une extrémité proximale de la tige ou du fil (20), dans laquelle l'unité d'entraînement (52) est configurée pour pousser la tige ou le fil (20) afin de fléchir l'élément flexible (10), et pour tirer la tige ou le fil (20) afin d'étendre l'élément flexible (10).

9. Orthèse dynamique selon la revendication 8, dans laquelle une longueur d'arc du sommet de l'élément flexible (10) augmente lorsque la tige ou le fil (20) est poussé tandis qu'une longueur d'arc du fond de l'élément flexible (10) reste inchangée.

10. Orthèse dynamique selon la revendication 1, dans laquelle les os du doigt comprennent des métacarpiens ou des phalanges, et dans laquelle la pluralité d'articulations comprend une articulation métacarpo-phalangienne et/ou une articulation interphalangienne proximale ou distale.

11. Orthèse dynamique selon la revendication 1, comprenant en outre un capteur (60), configuré pour mesurer une compression ou une tension de la tige ou du fil (20).

12. Orthèse dynamique selon la revendication 11, dans laquelle le capteur (60) comprend :
un conducteur Bowden (62), configuré pour mesurer des forces de compression et de traction appliquées à la tige ou au fil (20) par rapport à l'élément flexible (10) ; et
une structure en parallélogramme (64), configurée pour appliquer une résistance aux mouvements de flexion dans le conducteur Bowden (62).

13. Orthèse dynamique selon la revendication 1, dans laquelle l'élément flexible (10) comprend une plaque de verrouillage (13), configurée pour interconnecter une pluralité des unités interconnectées (12) afin de limiter ou de réduire un pliage d'une partie de l'élément flexible (10).

14. Orthèse dynamique selon la revendication 1, dans laquelle ledit élément flexible (10) présente une amplitude de mouvement de zéro degré à 250 degrés.

15. Orthèse dynamique selon la revendication 1, dans laquelle une partie (15) de l'élément flexible (10) comprend une rangée sous-jacente de nervures (19) qui forment une structure (24) qui se monte sur une partie terminale du doigt.
